# EUROPEAN PATENT APPLICATION

(11) **EP 1 361 285 A2**
(43) Date of publication of application: **12.11.2003**
(21) Application number: 02077062.4
(22) Date of filing: 28.05.2002
(51) Int. Cl.: C12Q 1/48

(54) **Protein kinase peptide substrate determination using peptide libraries**

(30) Priority: 30.05.2001 US 294365 P
(71) Applicant: SMITHKLINE BEECHAM CORPORATION, Philadelphia Pennsylvania 19103 (US)
(72) Inventor: Blackburn, Robert Kevin, c/o GlaxoSmithKline, Five Moore Drive, North Carolina 27709 (US); Bramson, Harold Neal, c/o GlaxoSmithKline, Five Moore Drive, North Carolina 27709 (US); Moyer, Mary Benbow, c/o GlaxoSmithKline, Five Moore Drive, North Carolina 27709 (US); Stuart, James Darren, c/o GlaxoSmithKline, Five Moore Drive, North Carolina 27709 (US)
(74) Representative: Connell, Anthony Christopher

(57) **Abstract**

A method of isolating and identifying peptide substrates for a protein kinase is disclosed. The method involves a combination of size exclusion and gallium-based metal affinity chromatography. The method includes the steps of incubating a protein kinase with a peptide library in the presence of kinase reaction components, the library comprising library members; separating library members from the kinase reaction components using size exclusion chromatography to give a pool of phosphopeptides and unphosphorylated peptides; contacting the pool with immobilized gallium ions to form chelated phosphopeptides; eluting chelated phosphopeptides away from the gallium ions to give eluted phosphopeptides; sequencing the eluted phosphopeptides, whereby a preferred amino acid sequence of a preferred peptide substrate for a protein kinase is elucidated. Also disclosed is a method of identifying a compound that modulates the protein kinase catalyzed phosphorylation of a peptide substrate and a method of designing protein kinase substrates.

SEQUENCE LISTING

<110> SmithKline Beecham Corporation

Stuart, J.

Bramson, H.

Moyer, Mary

Blackburn, Kevin

<120> PROTEIN KINASE PEPTIDE SUBSTRATE DETERMINATION USING PEPTIDE
LIBRARIES

<130> PU4253

<160> 17

<170> PatentIn version 3.0

<210> 1

<211> 15

<212> PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  1

Met Ala Xaa Xaa Xaa Xaa Tyr Xaa Xaa Xaa Xaa Ala Lys Lys Lys
1               5                   10                  15

<210>  2

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  2

Met Ala Xaa Xaa Xaa Xaa Ser Xaa Xaa Xaa Xaa Ala Lys Lys Lys
1               5                   10                  15

<210>  3

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  3

Met Ala Xaa Xaa Xaa Xaa Thr Xaa Xaa Xaa Xaa Ala Lys Lys Lys
1               5                   10                  15

<210>  4

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  4

Met Ala Xaa Xaa Xaa Xaa Ser Pro Xaa Xaa Xaa Ala Lys Lys Lys
1               5                   10                  15

<210>  5

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  5

Met Ala Xaa Xaa Xaa Xaa Thr Pro Xaa Xaa Xaa Ala Lys Lys Lys
1               5                   10                  15

<210>  6

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  6

Arg Ala Xaa Xaa Xaa Xaa Tyr Xaa Xaa Xaa Xaa Ala Lys Lys Lys
1               5                   10                  15

<210>  7

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  7

Arg Ala Xaa Xaa Xaa Xaa Ser Xaa Xaa Xaa Xaa Ala Lys Lys Lys
1               5                   10                  15

<210>  8

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  8

Arg Ala Xaa Xaa Xaa Xaa Thr Xaa Xaa Xaa Xaa Ala Lys Lys Lys
1               5                   10                  15

<210>  9

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  9

Arg Ala Xaa Xaa Xaa Xaa Ser Pro Xaa Xaa Xaa Ala Lys Lys Lys
1               5                   10                  15

<210>  10

<211>  15

<212>  PRT

<213>  Synthetic Peptide

<220>

<221>  PEPTIDE

<222>  (1)..(15)

<223>  Xaa is independently A, R, N, D, E, Q, G, H, I, L, K, M, F, P
or V

<400>  10

Arg Ala Xaa Xaa Xaa Xaa Thr Pro Xaa Xaa Xaa Ala Lys Lys Lys
1               5                   10                  15

<210>  11

<211>  22

<212>  PRT

<213>  Homo sapiens

<220>

<221>  PEPTIDE

<222>  (1)..(20)

<223>  N-terminus biotin conjugated, C-terminal amide grouped

<300>

<301>  Jan et al.

<302>  Insights into the HER-2 Receptor Tyrosine Kinase Mechanism and
Substrate Specificity Using a Transient Kinetic Analysis

<303>  Biochemistry

<304>  39

<305>  32

<306>  9786-9803

<307>  2000-07-20

<400>  11

Xaa Xaa Xaa Xaa Xaa Xaa Gly Gly Met Glu Asp Ile Tyr Phe Glu Phe
1               5                   10                  15

Met Gly Gly Lys Lys Lys
            20

<210>  12

<211>  15

<212>  PRT

<213>  Synthetic Peptide

<400>  12

Arg Ala His Glu Glu Ile Tyr His Phe Phe Phe Ala Lys Lys Lys
1               5                   10                  15

<210>  13

<211>  15

<212>  PRT

<213>  Synthetic Peptide

<400>  13

Arg Ala His His Glu Ile Tyr His Phe Phe Phe Ala Lys Lys Lys
1               5                   10                  15

<210>  14

<211>  15

<212>  PRT

<213>  Synthetic Peptide

<400>  14

Arg Ala His His Glu Glu Tyr His Phe Phe Phe Ala Lys Lys Lys
1               5                   10                  15

<210>  15

<211>  15

<212>  PRT

<213>  Synthetic Peptide

<400>  15

Arg Ala His Glu Glu Glu Tyr His Phe Ile Phe Ala Lys Lys Lys
1               5                   10                  15

<210>  16

<211>  15

<212>  PRT

<213>  Synthetic Peptide

<400>  16

Arg Ala His Glu Glu Glu Tyr His Phe Phe Phe Ala Lys Lys Lys
1               5                   10                  15

<210>  17

<211>  16

<212>  PRT

<213>  Synthetic Peptide

<400>  17

Gly Gly Met Glu Asp Ile Tyr Phe Glu Phe Met Gly Gly Lys Lys Lys
1               5                   10                  15

## Description

### Cross-Reference to Related Applications

This application claims priority to Provisional application number 60/294,365 filed 30 May 2001.

### Technical Field

The present invention relates generally to the identification of peptide substrates for protein kinases and more particularly to improved methods of purifying phosphorylated protein kinase substrates. The invention further relates to methods of identifying natural substrates for protein kinases and methods of using purified phosphorylated protein kinase substrates as templates for protein kinase modulator design.

| Abbreviations | |
|---|---|
| Ahx | aminohexyl |
| ATP | adenosine triphosphate |
| cDNA | complementary DNA |
| DNA | deoxyribonucleic acid |
| DTT | dithiothreitol |
| EGFR | epidermal growth factor receptor |
| Fmoc | 9-fluorenylmethoxycarbonyl |
| HPLC | high performance liquid chromatography. |
| HTRF | homogenous time-resolved fluorescence |
| IDA | iminodiacetic acid |
| IMAC | immobilized metal affinity chromatography |
| MCAC | metal chelate affinity chromatography |
| MOPS | 3-(N-morpholino)propanesulphonic acid |
| MES | 2-[N-morpholino]ethanesulfonic acid |
| PCR | polymerase chain reaction |
| PDGFR | platelet-derived growth factor receptor |
| PTH | phenylthiohydantoin |
| SDS | sodium dodecyl sulfate |
| SEC | size exclusion chromatography |
| SPA | scintillation proximity assay |
| tBoc | t-butyloxycarbonyl |
| TED | tris(carboxymethyl)-ethylenediamine |
| TFA | trifluoroacetic acid |

| Table of Amino Acid Abbreviations | | |
|---|---|---|
| Single-Letter Code | Three-Letter Code | Name |
| A | Ala | Alanine |
| V | Val | Valine |
| L | Leu | Leucine |
| I | lie | Isoleucine |
| P | Pro | Proline |
| F | Phe | Phenylalanine |
| W | Trp | Tryptophan |
| M | Met | Methionine |
| G | Gly | Glycine |
| S | Ser | Serine |
| T | Thr | Threonine |
| C | Cys | Cysteine |
| Y | Tyr | Tyrosine |
| N | Asn | Asparagine |
| Q | Gln | Glutamine |
| D | Asp | Aspartic Acid |
| E | Glu | Glutamic Acid |
| K | Lys | Lysine |
| R | Arg | Arginine |
| H | His | Histidine |

### Background Art

Protein phosphorylation is a common post-translational modification that regulates many important cellular processes such as metabolism, growth, and differentiation. Phosphorylation of a given amino acid in a protein can have a variety of effects, such as activating or inactivating a protein's enzymatic activity, or altering a protein's affinity for binding to other proteins. Protein phosphorylation occurs most commonly on three amino acids: tyrosine, serine and threonine, each of which contains a hydroxyl group.

During the phosphorylation event, a high-energy phosphoryl group is commonly transferred from an adenosine triphosphate molecule (ATP) to a particular protein via a protein kinase. Protein kinases can be broadly categorized based on the amino acids to which a phosphoryl group is transferred. For example, protein tyrosine kinases and protein serine/threonine kinases catalyze the phosphorylation of tyrosine and serine or threonine residues, respectively. Phosphorylation can occur in response to cellular signals originating from hormones, neurotransmitters, growth and differentiation factors, and other molecules. A phosphoryl group can be enzymatically removed from a phosphorylated protein by a protein phosphatase.

Given their key role in cellular regulation, it is not surprising that defects in protein kinases have been implicated in many disease states and conditions. Inappropriately controlled signaling has been implicated in a variety of disease conditions including inflammation, cancer, arteriosclerosis and psoriasis. For example, the overexpression of cellular tyrosine kinases such as the epidermal growth factor receptor (EGFR) or platelet-derived growth factor receptor (PDGFR), or the mutation of tyrosine kinases to produce constitutively active forms is present in many cancer cells. Druker et al., (1996) *Nat. Med.* 2: 561-66. Protein kinases are also implicated in inflammatory signals. Defective Ser/Thr kinase genes have been implicated in several diseases such as myotonic dystrophy as well as cancer, and Alzheimer's disease. Sanpei et al., (1995) *Biochem. Biophys. Res. Commun.* 212: 341-46; Sperber et al., (1995) *Neurosci. Lett.* 197: 149-53; Grammas et al., (1995) *Neurobiol. Aging* 16: 563-69; Govoni et al., (1996) *Ann. N.Y. Acad. Sci.* 777: 332-37.

Modulators of protein kinase activities are being pursued as therapeutically useful agents for the treatment of some diseases. Modulators of protein kinase activities can be identified by screening compounds in protein kinase assays. Protein kinase assays typically require a kinase, ATP, MgCl₂ or MnCl₂, and a substrate that is phosphorylated in the presence of the kinase. The substrate can be the natural protein substrate or a peptide substrate. A peptide substrate is often desired when using high-throughput screening formats such as homogenous time-resolved fluorescence (HTRF) or scintillation proximity assays (SPA).

At present, there are very few accurate and reliable methods for identifying protein kinase peptide substrates. In one currently available method in the art, one must first phosphorylate a fraction of a peptide library with a protein kinase of interest to form phosphopeptides and then isolate the resulting phosphopeptides using an iron ion-based affinity column for sequence determination. See, e.g., U.S. Patent Nos. 5,532,167 and 6,004,757, both to Cantley et al. This method appears to generate results that are not reproducible under all conditions.

The present invention modifies and extends a currently available method by employing a combination of size exclusion chromatography and gallium ion-based immobilized metal affinity chromatography for the isolation of phosphopeptides. The present invention discloses a simplified, accurate and reproducible method for isolating phosphopeptides identified as protein kinase substrates. The present inventive method offers a degree of speed and sensitivity heretofore unavailable in the art.

### Summary of the Invention

A method of elucidating a preferred amino acid sequence of a preferred peptide substrate for a protein kinase is disclosed. In a preferred embodiment, the method comprises: (a) incubating a protein kinase with a peptide library in the presence of kinase reaction components, the library comprising library members; (b) separating library members from the kinase reaction components using size exclusion chromatography to give a pool of phosphopeptides and unphosphorylated peptides; (c) contacting the pool with immobilized gallium ions to form chelated phosphopeptides; (d) eluting chelated phosphopeptides away from the gallium ions to give eluted phosphopeptides; (e) sequencing the eluted phosphopeptides, whereby a preferred amino acid sequence of a preferred peptide substrate for a protein kinase is elucidated.

A method of identifying a compound that modulates the protein kinase catalyzed phosphorylation of a preferred peptide substrate is also disclosed. In a preferred embodiment, the method comprises: (a) incubating a protein kinase with a peptide library in the presence of kinase reaction components, the library comprising library members; (b) separating library members from the kinase reaction components using size exclusion chromatography to give a pool of phosphopeptides and unphosphorylated peptides; (c) contacting the pool with immobilized gallium ions to form chelated phosphopeptides; (d) eluting chelated phosphopeptides away from the gallium ions to give eluted phosphopeptides; (e) sequencing the eluted phosphopeptides to elucidate a preferred amino acid sequence of a preferred peptide substrate for a protein kinase; (f) incubating a protein kinase with a preferred peptide substrate having the preferred amino acid sequence in the presence and absence of a test modulator compound; and (g) determining an amount of preferred peptide substrate that is phosphorylated in the presence of the protein kinase, wherein a difference in phosphorylation of the preferred peptide substrate in the presence of the test modulator compound relative to phosphorylation of the preferred peptide substrate in the absence of the test modulator compound is indicative of modulation, whereby a compound that modulates the protein kinase catalyzed phosphorylation of a preferred peptide substrate is identified.

A method of designing a modulator for a protein kinase additionally is disclosed. In a preferred embodiment, the method comprises: (a) incubating a protein kinase with a peptide library in the presence of kinase reaction components, the library comprising library members; (b) separating library members from the kinase reaction components using size exclusion chromatography to give a pool of phosphopeptides and unphosphorylated peptides; (c) contacting the pool with immobilized gallium ions to form chelated phosphopeptides; (d) eluting chelated phosphopeptides away from the gallium ions to give eluted phosphopeptides; (e) sequencing the eluted phosphopeptides to elucidate a preferred amino acid sequence of a preferred peptide substrate for a protein kinase, wherein the preferred peptide substrate is phosphorylated; (f) quantifying in an assay for protein kinase activity a degree to which the phosphorylated preferred peptide substrate comprising a preferred amino acid sequence modulates the activity of the protein kinase; (g) selecting a chemical modification of the phosphorylated preferred peptide substrate comprising a preferred amino acid sequence wherein the interaction between the protein kinase and the phosphorylated preferred peptide substrate is predicted to be modulated by the chemical modification; (h) performing the chemical modification on the phosphorylated preferred peptide substrate; (i) contacting the modified phosphorylated preferred peptide substrate with the protein kinase; (j) quantifying in a biological assay for protein kinase activity a degree to which the modified phosphorylated preferred peptide substrate modulates the biological activity of the protein kinase; and (k) comparing the activity of the protein kinase in the presence of the modified phosphorylated preferred peptide substrate with the activity of the protein kinase in the absence of the modified phosphorylated preferred peptide substrate, whereby a modulator for a protein kinase is designed.

A method of elucidating a preferred amino acid sequence of a preferred peptide substrate for a protein kinase is also disclosed. In a preferred embodiment, the method comprises: (a) incubating a protein kinase with a peptide library in the presence of kinase reaction components, the library comprising library members having one or more variant positions; (b) separating library members from the kinase reaction components using size exclusion chromatography to give a pool of phosphopeptides and unphosphorylated peptides; (c) contacting the pool with immobilized gallium ions to form chelated phosphopeptides; (d) eluting chelated phosphopeptides away from the gallium ions to give eluted phosphopeptides; (e) sequencing the eluted phosphopeptides; (f) fixing a residue at one or more variant positions in a library member; and (g) repeating steps (a) through (d) a desired number of times, whereby a preferred amino acid sequence of a preferred peptide substrate for a protein kinase is elucidated.

With respect to the foregoing embodiments of the present invention, the library members are preferably fifteen (15)-mers cleavably disposed on a support or on the surface of a phage. More preferably, the 15-mers have a sequence selected from the group consisting of M-A-X-X-X-X-Y-X-X-X-X-A-K-K-K (SEQ ID NO: 1), M-A-X-X-X-X-S-X-X-X-X-A-K-K-K (SEQ ID NO: 2), M-A-X-X-X-X-T-X-X-X-X-A-K-K-K (SEQ ID NO: 3), M-A-X-X-X-X-S-P-X-X-X-A-K-K-K (SEQ ID NO: 4), M-A-X-X-X-X-T-P-X-X-X-A-K-K-K (SEQ ID NO: 5), R-A-X-X-X-X-Y-X-X-X-X-A-K-K-K (SEQ ID NO: 6), R-A-X-X-X-X-S-X-X-X-X-A-K-K-K (SEQ ID NO: 7), R-A-X-X-X-X-T-X-X-X-X-A-K-K-K (SEQ ID NO: 8), R-A-X-X-X-X-S-P-X-X-X-A-K-K-K (SEQ ID NO: 9), and R-A-X-X-X-X-T-P-X-X-X-A-K-K-K (SEQ ID NO: 10), wherein X independently stands for any amino acid. More preferably still, X independently stands for A, R, N, D, E, Q, G, H, I, L, K, M, F, P or V. Yet more preferably, the 15-mers are present in equimolar amounts. Still more preferably, the peptides are amidated at the C-terminus. Even more preferably, the peptide components are lyophilized and resuspended before incubation with a protein kinase.

A method of designing a substrate for a protein kinase is furthermore disclosed. In a preferred embodiment, the method comprises: (a) providing an unmodified preferred peptide substrate selected from the group consisting of SEQ ID NOs. 1-10; (b) quantifying phosphorylation of the unmodified preferred peptide substrate in an assay measuring protein kinase activity; (c) selecting a chemical modification of the preferred peptide substrate wherein the interaction between the protein kinase and the unmodified preferred peptide substrate is predicted to be modulated by the chemical modification; (d) performing the chemical modification on the unmodified preferred peptide substrate to form a modified preferred peptide substrate; (e) contacting the modified preferred peptide substrate with the protein kinase; (f) quantifying phosphorylation of the modified preferred peptide substrate, in an assay measuring protein kinase activities; (g) comparing the activities of the protein kinase in the presence of the modified preferred peptide substrate with the activity of the protein kinase in the presence of the unmodified preferred peptide substrate; and (h) repeating steps (c) through (g) a desired number of times to achieve a desired substrate property, whereby a substrate for a protein kinase is designed.

Accordingly, it is an object of the present invention to provide the identification of peptide substrates for protein kinases. This and other objects are achieved in whole or in part by the present invention.

An object of the invention having been stated hereinabove, other objects will be evident as the description proceeds, when taken in connection with the accompanying Drawings and Examples as best described hereinbelow.

### Brief Description of the Drawing

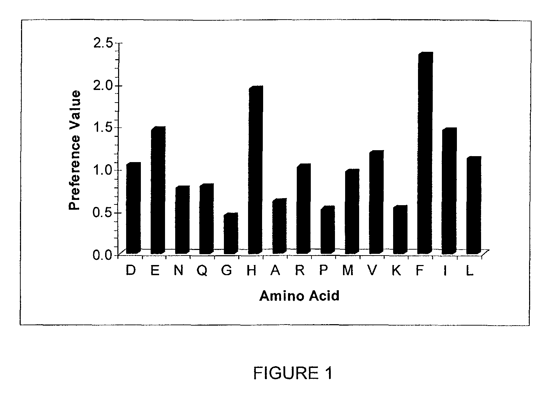

Figure 1 is a representative histogram depicting the amino acid preferences of ErbB-2 at one of the variable positions of a 15-mer peptide substrate.

### Detailed Description of the Invention

Until disclosure herein of the present invention, the ability to reliably and reproducibly screen a peptide library to identify a preferred amino acid sequence of a peptide substrate for a protein kinase has not been accurately realized. And until disclosure herein of the present invention, a reproducible and reliable method of screening a library of candidate substrates for their ability to function as kinase substrates based on the affinity of phosphorylated peptides for gallium has been unachieved.

### l. Definitions

Following long-standing patent law convention, the terms "a" and "an" mean "one or more" when used in this application, including the claims.

As used herein, the term "active site" means that site in an enzyme, for example a protein kinase, at which substrate binding occurs.

As used herein, the term "agonist" means an agent that supplements or potentiates the bioactivity of a functional gene or protein, or of a peptide encoded by a gene that is up- or downregulated by a peptide and/or by a gene that contains a binding site in its promoter region.

As used herein, the term "Ahx" means aminohexyl and refers to a 6-carbon linker sequence.

As used herein, the term "amino acid sequence" means an oligopeptide, peptide, polypeptide, or protein sequence, and fragments thereof, and to naturally occurring or synthetic molecules. Peptides are preferably about 5 to about 25 amino acids in length, and more preferably about 15 amino acids in length, i.e. a "15-mer." Where "amino acid sequence" is recited herein the term refers to an amino acid sequence of a synthetic peptide or a naturally occurring protein molecule or fragment thereof. The term "amino acid sequence" is not meant to limit an amino acid sequence to a complete, native amino acid sequence associated with a recited protein molecule.

As used herein, the term "antagonist" means an agent that decreases or inhibits the bioactivity of a functional gene or protein, or that supplements or potentiates the bioactivity of a naturally occurring or engineered non-functional gene or protein. Alternatively, an antagonist can decrease,or inhibit the bioactivity of a functional gene, or of a polypeptide encoded by a gene that is up- or down-regulated by a peptide and/or contains a binding site in its promoter region.

As used herein, the term "biologically active" means a protein having structural, regulatory, or biochemical functions of a naturally occurring molecule, whether the protein be a natural, recombinant, or fragment thereof.

As used herein, the term "chemical modification" means alteration of a first moiety by covalently or ionically binding a second moiety to the first moiety. Chemical modification can involve the addition of a detectable moiety to a peptide or protein. Chemical modification can also refer to the catalyzed or uncatalyzed addition of a phosphoryl group to a peptide.

As used herein, the terms "candidate substrate", "candidate protein kinase substrate", and "peptide substrate" are used interchangeably and mean a sample suspected of having, or actually having, the propensity to accept a phosphoryl group transferred from a donor. As used herein, the terms are used in their broadest sense and refer generally to a substance that is believed to interact with another moiety, for example a peptide that is believed to interact with a complete, or a fragment of, a protein kinase, and which can be subsequently evaluated for such an interaction.

As used herein, the terms "candidate compound", "candidate modulator" and "candidate substance" are used interchangeably and mean a compound or substance suspected of having, or actually having, the ability to modulate the transfer of a phosphoryl group. Such compounds can inhibit or induce the transfer of a phosphoryl group. Representative candidate compounds include xenobiotics such as drugs and other therapeutic agents, carcinogens and environmental pollutants, natural products and extracts, as well as endobiotics such as steroids, fatty acids and prostaglandins. Other examples of candidate compounds that can be investigated using the methods of the present invention include, but are not restricted to, agonists and antagonists of a peptide, toxins and venoms, viral epitopes, hormones (e.g., opioid peptides, steroids, etc.), hormone receptors, peptides, enzymes, enzyme substrates, co-factors, lectins, sugars, oligonucleotides or nucleic acids, oligosaccharides, proteins, small molecules and monoclonal antibodies.

As used herein, the term "detecting" means confirming the presence of a given target entity by observing the occurrence of a detectable signal, such as a radiologic or spectroscopic signal, that will appear exclusively in the presence of the target entity.

As used herein, the term "gene" is used for simplicity to refer to a functional protein, polypeptide or peptide encoding unit. As will be understood by those in the art, this functional term includes both genomic sequences and cDNA sequences.

As used herein, the term "interact" means detectable interactions between molecules, such as can be detected using, for example, a yeast two hybrid assay. The term "interact" is also meant to include "binding" interactions between molecules and non-covalent associations between molecules. Interactions can, for example, be protein-protein or protein-nucleic acid in nature.

As used herein, the term "isolated" means oligonucleotides substantially free of other nucleic acids, proteins, lipids, carbohydrates or other materials with which they can be associated, such association being either in cellular material or in a synthesis medium. The term can also be applied to polypeptides, in which case the polypeptide will be substantially free of nucleic acids, carbohydrates, lipids and other undesired polypeptides.

As used herein, the term "kinase assay" is used interchangeably with the term "kinase reaction" and means an enzyme-catalyzed chemical reaction wherein a phosphoryl group is transferred from a first moiety to a second moiety.

As used herein, the term "labeled" means the attachment of a moiety, capable of detection by spectroscopic, radiologic or other methods, to a probe molecule.

As used herein, the term "modified" means an alteration from an entity's normally occurring state. An entity can be modified by removing discrete chemical units or by adding discrete chemical units. The term "modified" encompasses detectable labels as well as those entities added as aids in purification.

As used herein, the term "modulate" means an increase, decrease, or other alteration of any or all chemical and biological activities or properties of a peptide or other molecule. The term "modulation" as used herein refers to both upregulation (i.e., activation or stimulation) and downregulation (i.e. inhibition or suppression) of a response.

As used herein, the term "mutation" carries its traditional connotation and means a change, inherited, naturally occurring or introduced, in a nucleic acid or polypeptide sequence, and is used in its sense as generally known to those of skill in the art.

As used herein, the term "phosphoryl group" means a discrete chemical unit comprising at least one phosphorus atom and three oxygen atoms. Phosphoryl groups can be transferred as a group from one entity to another entity.

As used herein, the terms "phosphopeptide", "phosphorylated candidate substrates" and "phosphorylated species" are used interchangeably and mean a peptide or protein that has been phosphorylated. That is, a peptide or protein to which a phosphoryl group has been transferred from a donor. The donor can be any type of compound, including nucleotide triphosphates and other phosphopeptides.

As used herein, the term "polypeptide" means any polymer comprising any amino acids, regardless of its size. Although "protein" is often used in reference to relatively large polypeptides, and "peptide" is often used in reference to relatively small polypeptides, usage of these terms in the art overlaps and varies. Thus, the term "polypeptide", as used herein, refers to peptides, polypeptides and proteins, unless otherwise noted. As used herein, the terms "protein", "polypeptide" and "peptide" are used interchangeably herein when referring to a gene product.

As used herein the term "preferred amino acid sequence" means an amino acid sequence that is preferentially or selectively recognized by a protein kinase, i.e. there is a preferential or selective interaction between the protein kinase and the amino acid sequence such that the amino acid sequence is readily phosphorylated. Correspondingly, the term "preferred peptide substrate" means a peptide substrate comprising the preferred amino acid sequence.

As used herein, the term "protein kinase" means a protein, namely an enzyme, capable of catalyzing the transfer of a phosphoryl group from a donor to an acceptor. In the context of the present invention, a protein kinase refers to a substantially purified protein kinase obtained from any species, preferably mammalian, including bovine, ovine, porcine, murine, equine, and more preferably human, from any source whether natural, synthetic, semisynthetic or recombinant.

As used herein, the term "sequencing" and grammatical derivations thereof means determining the ordered linear sequence of nucleic acids or amino acids of a DNA or peptide (or protein) target sample, using conventional manual or automated laboratory techniques.

As used herein, the term "variant" means an amino acid sequence, particularly an amino acid sequence of the present invention, which is altered by one or more amino acids. As noted further herein, the variant can have "conservative" changes, wherein a substituted amino acid has similar structural or chemical properties, e.g., replacement of leucine with isoleucine. More rarely, a variant can have "nonconservative" changes, e.g., replacement of a glycine with a tryptophan. Analogous minor variations can also include amino acid deletions or insertions, or both. Guidance in determining which amino acid residues can be substituted, inserted, or deleted without abolishing biological or immunological activity can be found using computer programs known to those of skill in the art, for example, DASTARD software. The term "variant" can be used interchangeably with the term "mutant".

### II. General Considerations

Immobilized metal affinity chromatography (IMAC), also known as metal chelate affinity chromatography (MCAC), is a specialized aspect of affinity chromatography. The principle behind IMAC lies in the fact that many transition metal ions (e.g., zinc and copper) can coordinate with amino acids via electron donor groups on the amino acid side chains. To utilize this interaction for chromatographic purposes, the metal ion (for example, gallium) is preferably immobilized on an insoluble support. Immobilization can be accomplished by attaching a chelating group to a chromatographic matrix, such as agarose or that sold under the registered trademark SEPHAROSE® by Pharmacia Fine Chemicals, Inc. of Piscataway, New Jersey. Most importantly, to be useful, the metal of choice preferably has a higher affinity for the matrix than for the compounds to be purified. Additionally, the metal of choice preferably exhibits reproducible binding profiles for a given peptide. This is helpful in removing experiment-to-experiment variations in binding.

A common chelating group used in this technique is iminodiacetic acid (IDA). It can be coupled to a matrix such as agarose or that sold under the registered trademark SEPHAROSE® by Pharmacia Fine Chemicals, Inc. of Piscataway, New Jersey, via a long spacer arm. The spacer arm ensures that the chelating metal is accessible to available binding sites, preferably all available binding sites, on a protein. Another popular chelating group for IMAC applications is tris(carboxymethyl)-ethylenediamine (TED). Appropriate metals for IMAC can include, but are not limited to, zinc, copper, nickel, cobalt, calcium, iron and gallium. See, e.g., Posewitz & Tempst. (1999) *Anal. Chem.* 71(14): 2883-92.

In practice, IMAC relies on metal ions immobilized on a matrix. Preparation of an IMAC column (or other structure) thus comprises attaching metal ions, such as gallium, to a matrix, such as IDA. Methods for these attachments are disclosed herein and will be apparent to those of skill in the art upon consideration of the present disclosure. Additional procedures, such as column packing and column equilibration, can be performed as required.

The development of IMAC as an aid in peptide, phosphopeptide and protein purification processes can be significantly simplified by accurate prediction of the protein affinity of a given protein for IMAC resins before performing separations in the laboratory. If the affinity for an IMAC resin could be reliably and easily predicted from its protein structure, then the researcher would be better informed when deciding on a development strategy. A protein predicted to have a high affinity, for example, could be bound to a resin under relatively stringent conditions and eluted with a simple isocratic step. Furthermore, a protein of only moderate affinity requires a less stringent binding condition and a sophisticated gradient elution. To achieve high resolution and maximum recovery in IMAC, however, a basic knowledge of the relative affinity of a species of peptide, phosphopeptide or proteins for a given immobilized metal is a desirable starting point.

### III. Procedure for Identifying Protein Kinase Peptide Substrates

In one aspect of the present invention, a method is disclosed whereby it is possible to reproducibly identify the amino acid sequence of a peptide substrate for a protein kinase. The identified peptide substrates can be used to measure protein kinase activity in assays for novel kinase inhibitors. Additionally, the identification of a peptide substrate sequence recognized by a protein kinase can aid in the identification of a natural protein substrate of a protein kinase of interest.

### III.A Preparation of Candidate Protein Kinase Substrates

Candidate protein kinase substrates suitable for use in the present invention are preferably peptides. Peptides of any length or amino acid composition can be used as candidate substrates. In a preferred embodiment, candidate protein kinase substrates are 15-mers, i.e. peptides having 15 amino acid residues. In a more preferred embodiment, candidate protein kinase substrates have a sequence selected from among M-A-X-X-X-X-Y-X-X-X-X-A-K-K-K (SEQ ID NO: 1), M-A-X-X-X-X-S-X-X-X-X-A-K-K-K (SEQ ID NO: 2), M-A-X-X-X-X-T-X-X-X-X-A-K-K-K (SEQ ID NO: 3), M-A-X-X-X-X-S-P-X-X-X-A-K-K-K (SEQ ID NO: 4), M-A-X-X-X-X-T-P-X-X-X-A-K-K-K (SEQ ID NO: 5), R-A-X-X-X-X-Y-X-X-X-X-A-K-K-K (SEQ ID NO: 6), R-A-X-X-X-X-S-X-X-X-X-A-K-K-K (SEQ ID NO: 7), R-A-X-X-X-X-T-X-X-X-X-A-K-K-K (SEQ ID NO: 8), R-A-X-X-X-X-S-P-X-X-X-A-K-K-K (SEQ ID NO: 9), and R-A-X-X-X-X-T-P-X-X-X-A-K-K-K (SEQ ID NO: 10), wherein X independently stands for A, R, N, D, E, Q, G, H, I, L, K, M, F, P, or V. In this embodiment, the permutations of preferred amino acids and variant positions permit preparation of up to 2.56 x 10⁹ unique peptides. In an even more preferred embodiment, the individual candidate protein kinase substrates are present in equimolar amounts. Optionally, the C-terminus of a peptide is substituted with an amide group, i.e. is amidated.

In the above sequences, and throughout the present disclosure, X denotes a variant position in a peptide sequence. That is, X denotes a position in the sequence of a peptide at which the identity of an amino acid can independently vary. The term "variant position," therefore, is used to refer to positions in a peptide sequence (e.g. a library member) at which the identity of an amino acid can independently vary. A single sequence can comprise one or more variant positions.

A residue can also be fixed at a variant position in a peptide sequence comprising one or more variant positions. That is, a residue can be incorporated at a variant position and fixed at that position, effectively making the variant position an invariant position. Thus, fixing a residue at a variant position means selecting a residue to thereafter occupy a variant position such that the identitiy of the residue at the variant position does not change. One or more than one variant position(s) can have a residue fixed at that variant position.

The effect of phosphorylating one residue in a kinase substrate can affect the degree of phosphorylation of residues at other sites in the substrate. Therefore, by fixing a phosphorylated residue at a variant position in a substrate, such as those disclosed herein, the effect of the phosphorylated residue on the degree of phosphorylation of other residues in the substrate can be assessed. For example, a phosphotyrosine, phosphoserine or phosphothreonine can be fixed at a variant position in a substrate and the effect of the phosphorylated residue can then be assessed.

The process of fixing a residue at a variant position can be employed in a process of substrate design in accordance with the present invention and can be employed in an iterative refinement strategy. For example, a phosphotyrosine can be positioned at a variant position in a substrate. The effect of the phosphotyrosine on the degree of phosphorylation of other residues of the substrate can then be assessed. Subsequently, another phosphorylated residue can be fixed at another variant position and the effect of more than one phosphorylated residue on the phosphorylation of other residues of the substrate can be assessed.

A candidate protein kinase substrate can be generated by a number of methods. For example, DNA coding for a given candidate peptide substrate can be expressed and purified using methods known to those of ordinary skill in the art. Phage display represents one non-limiting example; see, e.g. U.S. Patent No. 5,260,203, herein incorporated by reference. Standard recombinant DNA and molecular cloning techniques can also be employed. Exemplary, non-limiting methods are described by Sambrook et al., (eds.) (1989) Molecular Cloning, Cold Spring Harbor Laboratory Press, Cold Spring Harbor, New York; by Silhavy et al., (1984) Experiments with Gene Fusions, Cold Spring Harbor Laboratory Press, Cold Spring Harbor, New York; by Ausubel et al., (1992) Current Protocols in Molecular Biology, John Wylie and Sons, Inc., New York; and by Glover, (ed.) (1985) DNA Cloning: A Practical Approach, MRL Press, Ltd., Oxford, U.K.

Alternatively, larger proteins comprising an identified candidate protein kinase substrate can be subjected to proteolytic degradation to excise the identified substrate, which can subsequently be purified. Finally, and most preferably, a candidate substrate can be synthesized using standard peptide synthetic techniques, which will preferably involve automated peptide synthesis using a commercially available peptide synthesizer, such as the Model 348 peptide synthesizer, available from Advanced ChemTech, Inc., of Louisville, Kentucky.

Synthetic chemistry techniques, such as t-butyloxycarbonyl (tBoc) or 9-fluorenylmethoxycarbonyl (Fmoc) synthesis, are preferred for reasons of purity, antigenic specificity, freedom from undesired side products, ease of production and the like. A summary of the many techniques available can be found in Steward et al., (1969) Solid Phase Peptide Synthesis, W. H. Freeman Co., San Francisco; Bodanszky et al., (1976) Peptide Synthesis, Second Edition, John Wiley & Sons Inc., New York; Meienhofer, (1983) Hormonal Proteins and Peptides, Vol. 2, p. 46, Academic Press, New York; Merrifield, (1969) *Adv. Enzymol,* 32:221-96; Fields et al., (1990) *Int. J.* *Peptide Protein Res.,* 35:161-214; and U.S. Patent No. 4,244,946 to Rivier et al. for solid phase peptide synthesis, each of which is incorporated herein by reference. Schroder et al. (Schroder et al., (1965) The Peptides, Vol. 1, Academic Press, New York) present a discussion of classical solution synthesis, which is incorporated herein by reference. Appropriate protective groups usable in such synthesis are described in the above texts and in McOmie, (1973) Protective Groups in Organic Chemistry, Plenum Press, New York, New York, which is incorporated herein by reference.

In general, the solid-phase synthesis methods referenced above comprise the sequential addition of one or more amino acid residues or suitably protected amino acid residues to a growing peptide chain. Normally, a suitable, selectively removable protecting group protects either the amino or carboxyl group of the first amino acid residue. A different, selectively removable protecting group is utilized for amino acids containing a reactive side group such as lysine.

Using a solid phase synthesis as exemplary, the protected or derivatized amino acid is attached to an inert solid support through its unprotected carboxyl or amino group. The protecting group of the amino or carboxyl group is then selectively removed and the next amino acid in the sequence having the complimentary (amino or carboxyl) group suitably protected is admixed and reacted under conditions suitable for forming the amide linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence, any remaining terminal and side group protecting groups (and solid support) are removed sequentially or concurrently, to generate the final linear polypeptide.

In one embodiment, candidate protein kinase substrates can be synthesized and bound to a solid support. Alternatively, a linker arm can be affixed to a candidate protein kinase substrate, either as a part of or subsequent to synthesis of the candidate substrate, and used to tether the candidate substrate to a solid support. In another embodiment, a candidate protein kinase substrate can be synthesized directly on a solid support, such as plastic pins, flat plates or welled plates.

In a more preferred embodiment a plurality of non-redundant candidate protein kinase substrates are synthesized using an automated peptide synthesizer, as noted above. The generated candidate protein kinase substrates are then pooled together in a single vessel as a mixture, purified by chromatography (e.g., size exclusion chromatography), lyophilized, and stored desiccated at -80°C to -20°C until needed. Solutions of the candidate protein kinase substrates can be made by dissolving the lyophilized powder in H₂O, or an aqueous buffer, and stored at -80°C to -20°C until needed. The concentration of candidate protein kinase substrates in this solution is preferably determined by amino acid analysis using the Waters Millipore ACCQ-TAG^{TM} method (Waters Corp., Milford, Massachusetts). Based on the volume of sample analyzed and the total yield of amino acids, the concentration can be calculated using the average molecular weight of the individual candidate substrates in the library. The collection of candidate protein kinase substrates can be referred to as a "library" or a "peptide library".

### III.B. Protein Kinase Phosphorylation Reaction

In one aspect of the present invention, a kinase reaction is performed in order to evaluate candidate protein kinase substrates (which are preferably peptide library members) for their ability to interact with, and be phosphorylated in the presence of, a protein kinase of interest. Needless to say, the selected protein kinase, or a domain or region thereof, exhibits phosphotransferase activity.

The components of a kinase assay of the present invention preferably comprises two primary components. First, a kinase component that exhibits phosphorylation activity is included. The kinase component is preferably of a concentration known to be optimal for producing a desired level of total peptide library phosphorylation within a reasonable time period. The actual concentration of a kinase component can vary, depending on the specific activity of the kinase of interest when the peptide library is used as the source of substrates.

Second, an assay buffer is included. In a preferred embodiment, the assay buffer can comprise 1-10 mM MgCl₂ or MnCl₂, 1 mM ATP, 0.2 mCi/mL [γ-³³P]ATP and about 2 mM of candidate protein kinase substrates, which can be prepared as described herein. The total volume of the assay mixture is preferably about 0.4 mL. The pH of the reaction mixture, after including all components, should be an optimum pH for the kinase of interest, which is commonly around pH 7.5. Although the candidate protein kinase substrates are preferably prepared using methods disclosed herein, other methods known to those of skill in the art may also be employed.

After preparing a suitable assay buffer and providing a protein kinase of interest, a kinase assay can be performed generally as follows. A kinase component, which is preferably a protein kinase, is added to the assay buffer and incubated for a predetermined period of time. The incubation time is selected so to preferably allow between 0.4% and 1.0% of a peptide library to be phosphorylated.

Experiments involving kinase reactions wherein a kinase component was omitted indicate that about 0.1-0.3% of a peptide library binds to a gallium-IDA matrix and elutes with the phosphopeptides, as described herein. Therefore, in general at least 0.2 - 0.4% of a peptide library should be phosphorylated to overcome this contamination by unphosphorylated peptides. The percentage of a peptide library that is phosphorylated is preferably kept at a minimum for the most effective evaluation of preferred peptide substrate sequences.

The incubation time of a kinase reaction (i.e. the length of time over which a kinase component is incubated with one or more candidate protein kinase substrates) is dependent on the concentration of protein kinase substrates in the peptide library and the enzyme turnover number with these substrates. The amount of time necessary to phosphorylate a desired percentage of the peptide library can be determined by analysis of reaction aliquots using established techniques, such as phosphocellulose filter binding assays.

At the conclusion of the incubation time, any phosphorylation reaction occurring at that time is halted. A preferred method of halting the phosphorylation reaction is by adding glacial acetic acid to the reaction mixture to a final concentration of about 5.5%. The volume of acetic acid added is preferably kept to about 5.5% of the original assay volume in order to minimize the dilution of candidate protein kinase substrates in the mixture.

### III.C. Isolation of Phosphorylated Peptides

After halting any kinase reaction occurring, it is necessary to isolate peptides that were phosphorylated in the presence of the protein kinase. Broadly, this is a two-step process. First, it is necessary to separate the phosphorylated and unphosphorylated peptides away from the other components in the protein kinase reaction mixture. Second, it is necessary to separate the phosphorylated protein kinase substrates (peptides that were recognized by the protein kinase of interest and, consequently, phosphorylated) from unphosphorylated peptides (peptides that were not recognized by the protein kinase as substrates and, consequently, not phosphorylated). The particularities of this two-step isolation procedure is one aspect of the present invention that offers an advantage over prior art separation methods.

### III.C.1.Size Exclusion Chromatography

Initially, both phosphorylated and unphosphorylated peptides are separated from the components of the protein kinase reaction mixture. In order to separate phosphorylated and unphosphorylated peptides from the other components of the assay, size exclusion chromatography is preferably employed.

In size exclusion chromatography (SEC), a mixture of entities having different molecular weights and therefore different spatial sizes and shapes are introduced to an SEC matrix. Preferably, the SEC matrix comprises bead-like structures having cavitous pore-like inclusions. A suitable SEC matrix can be selected based on the size of the pores disposed on the beads of the matrix. When entities are passed over the SEC matrix, smaller entities can enter the pores, while larger entities will pass between the beads. Due to the differences in the effective volumes of eluant components, small entities can enter the pores and be retained for a time; larger entities cannot enter the pores and thus, large entities elute earlier in the elution profile. Typically, the SEC matrix is packed in a vertical column. A sample is applied to the top of the column, and eluted material is collected at the bottom of the column.

As applied to the present invention, size exclusion chromatography is used to separate phosphorylated and unphosphorylated peptides from the components of the kinase reaction mixture. In a preferred embodiment, the SEC matrix is SUPERDEX^{TM} HR 10/30 (Amersham Pharmacia Biotech, United Kingdom), which is packed in a column suitable for use with a high performance liquid chromatography system (HPLC) system. See generally, Janson & Rydén (eds), (1998) Protein Purification: Principles, High Resolution Methods, and Applications (2^{nd} ed.), Wiley-Liss, New York and Hagel, (1993) *J. Chromatogr.* 648:19. Preferably, the column has bed dimensions of 10 mm by 300 to 310 mm, with a corresponding bed volume of about 24 mL. Suitable HPLC systems include, for example, the Dynamax Model SD-200 Solvent Delivery System, available from the Rainin Instrument Company, Inc. of Woburn, Massachusetts.

In a preferred method of separating phosphopeptides and unphosphorylated peptides from other kinase reaction components using HPLC, a volume of about 0.3 mL of the halted reaction mixture is applied to the HPLC column in a series of 0.1 - 0.15 mL injections. After each. injection a volume of mobile phase is applied to the column. A preferred mobile phase comprises 20% acetonitrile, 0.1% trifluoroacetic acid (TFA). The flow rate through the column can be maintained at a desired level, such as 0.6 mL/min. Eluted peptides are collected using a fraction collector. The presence of peptides is detected by spectrophotometrically observing each fraction at wavelengths of 210 and 280 nm. Baseline separation of the phosphorylated and unphosphorylated peptides from the other components of the protein kinase assay is achievable. Fractions containing detectable amounts of peptides are pooled, frozen in lyophilization vessels and lyophilized to dryness. Lyophilized peptides can be stored at -80°C to -20°C.

### lll.C.2.Immobilized Metal Affinity Chromatography

Following isolation of phosphorylated and unphosphorylated peptides from the other protein kinase assay mixture components, phosphorylated peptides (e.g. phosphopeptides) are separated from unphosphorylated peptides. This separation preferably employs gallium-based metal affinity chromatography and offers an advantage of the present invention over the prior art.

Preliminarily, lyophilized peptides (such as those described above) can be resuspended in a buffer solution. A preferred volume of buffer is about 0.8 mL. Suitable buffers will be apparent to those of skill in the art, and a preferred embodiment comprises 50 mM 2-[N-morpholino]ethanesulfonic acid (MES), and 1 M NaCI, pH = 5.5. Next, a iminodiacetic acid (IDA) column (Pierce Chemical Co., Bedford, Massachusetts) with a 0.5 mL bed volume is prepared. Iminodiacetic acid chelates metal ions, and is known to those of skill in the art of metal affinity chromatography. Gravity flow is used throughout the metal affinity chromatography method and a column can be prepared as follows.

A chromatography column of any size can be used to form the IDA column, but will preferably be a 5 mL polypropylene column (Pierce Chemical Co., Bedford, Massachusetts). The column is oriented vertically with a porous polyethylene disc placed inside the bottom end and sealed at the bottom by a removable cap. About 4 mL of water is added to the column and then the bottom cap is removed while simultaneously adding 1 mL of a 50 percent (v/v) slurry of IDA (Pierce Chemical Co., Bedford, Massachusetts). The water is allowed to flow out of the column and then another porous polyethylene disc is placed on top of the remaining 0.5 mL of packed IDA resin.

The column is oriented vertically and washed with at least 5 mL of water and subsequently charged with a gallium salt, preferably gallium chloride (GaCl₃). Preferably, the described column is charged with 5 mL of 22.7 mM GaCl₃. Other gallium salts would be apparent to one of ordinary skill in the art after a review of the disclosure of the present invention presented herein.

The charged column is then washed with water (at least about 8 mL for the described 5 mL column) and subsequently equilibrated with an appropriate buffer. Preferably, the equilibration buffer is the same buffer used to resuspend the lyophilized peptides. Thus, if the MES buffer described above is used to resuspend the lyophilized peptides, the same MES buffer is preferably used to equilibrate the column. The use of the same buffer eliminates a source of potential impurities and complications in the isolation process.

The resuspended peptide sample is then applied to the charged gallium column. The peptide sample can be applied by introducing the sample to the top of the column while simultaneously or subsequently drawing the sample into the matrix via gravity flow. The column is washed with a sufficient amount (at least about 10 mL for the described 5 mL column) of a suitable buffer (e.g. same MES buffer used for column equilibration) to draw the peptides into the column matrix and into contact with gallium ions associated with the IDA moieties.

The affinity of gallium for the phosphopeptides causes them to be retained on the column by the formation of a complex between the phosphoryl group of the phosphopeptides and the gallium ions disposed on the column matrix. Unphosphorylated peptides, which lack a phosphate group, preferably will not strongly associate with the gallium ions and will wash through the column. Finally, complexed phosphopeptides can be eluted away from the gallium ions using a suitable volume of an appropriate elution buffer solution to generate a pool of phosphopeptides. Preferably, the elution buffer comprises 500 mM ammonium bicarbonate, pH = 8.0 and about 4 mL of elution buffer is used to elute the phosphopeptides from the gallium column for the described 5 mL column.

### III.D. Sequence Determination

Pooled phosphopeptides are then subjected to peptide sequencing to determine the amounts of each amino acid present at each variable position of the mixture of peptide substrate sequences (I.e. positions denoted by "X" in SEQ ID NOs: 1-10). Standard peptide sequencing techniques can be employed, such as Edman degradation. See, e.g., Hunkapillar et al., (1984) *Science* 226: 304-11; Aebersold et al., (1990) *Anal. Biochem.* 187: 56-65; Hewick et al., (1981) *J. Biol. Chem.* 256: 7990-97; Konigsberg & Steinman, (1977) in The Proteins (3^{rd} ed.) (Neurath & Hill, eds.) 3: 1-178 and Stein & Udenfriend, (1984) *Anal. Chem.* 136:7-23. Automated peptide sequencers, such as the Hewlett-Packard G1005A Protein Sequencer (Hewlett-Packard Co., Palo Alto, California), are particularly useful in this aspect of the present invention.

Phosphopeptide sequence data can be presented as an overall indication of the amino acids present in the pool of phosphopeptides and, importantly, as a composite map of the relative amounts of each amino acid present in a phosphopeptide pool. Because a pool of phosphopeptides might not be homogeneous with respect to one particular peptide species, the sequence data is preferably interpreted and presented as a distribution of peptide sequences recognized as peptide substrates by a protein kinase.

### III.E. Interpretation of Phosphopeptide Sequence Data

A strength of the present inventive method is its ability to predict primary sequences of peptides (i.e. preferred amino acid sequences) recognized as substrates (i.e. preferred peptide substrates) by a given protein kinase. Phosphopeptide sequence data can be used as a general indicator of what representative sequences are recognized by a given protein kinase. Thus, general conclusions about the amino acid sequence requirements for a peptide substrate can be drawn from a generated amino acid composition profile. These conclusions can be used as a guide to identify one or more preferred peptide substrates for a given protein kinase.

### IV. Applications of the Present Invention

The present invention finds application in a number of experimental settings. Primarily, the present invention facilitates the elucidation of amino acid sequence requirements of a peptide (i.e. a preferred amino acid sequence), in order for the peptide to be recognized by a given protein kinase as a substrate (i.e. a preferred peptide substrate). This information can be extrapolated to learn more about the catalytic site of a protein kinase. For example, if the phosphopeptide amino acid composition trend indicates the presence of numerous charged amino acids, this information can be indicative of the presence of charged residues in the active site of a corresponding protein kinase. Similarly, if the phosphopeptide amino acid composition trend indicates the presence of uncharged hydrophobic amino acids, the active site of the corresponding protein kinase might comprise hydrophobic residues.

Additionally, the sequence information obtained from the present invention can be used to design modulators of protein kinases. Furthermore, the sequence information obtained from the present invention can be used to assist in the identification of one or more natural substrates of a given protein kinase. Each of these applications of the present invention is described herein below. The present invention can also be used in the development of peptide mimetics.

### IV.A. Development of Protein Kinase Modulators

Peptide substrates for a given protein kinase identified using the present invention can be useful in the development of protein kinase inhibitors. Specifically, identified peptide substrates (i.e. peptides preferred as substrates by a protein kinase) can be used to measure protein kinase activity in assays for novel protein kinase modulators.

When developing enzyme modulator compounds, there is a requirement that a suitable enzyme activity assay procedure be available to assess the effectiveness of a candidate modulator. Typically, the effectiveness of a candidate modulator will be judged in an assay that measures enzyme activity using a known substrate. For example, a ligand known to bind a protein kinase, such as a substrate exhibiting sequence properties identified using the present invention, can be used in an assay method as the ligand that is phosphorylated in the presence of a kinase where the inhibition or enhancement of phosphorylation by a test compound is gauged. A preferred method of determining whether a test compound is an inhibitor of a protein kinase of interest can comprise (a) incubating a protein kinase with a substrate identified using the present invention, which is known to interact with the protein kinase, in the presence of a test inhibitor compound; (b) determining an amount of substrate that is phosphorylated in the presence of the protein kinase, wherein decreased phosphorylation of the substrate in the presence of the protein kinase and the test inhibitor compound relative to phosphorylation in the absence of the test inhibitor compound is indicative of inhibition; and (c) identifying the test compound as an inhibitor of phosphorylation if decreased phosphorylation is observed.

Similarly, the present invention can be used to identify an agonist of a protein kinase. A preferred method of determining whether a test compound is an agonist of a protein kinase of interest can comprise (a) incubating a protein kinase with a substrate identified using the present invention, which is known to interact with the protein kinase, in the presence of a test agonist compound; (b) determining an amount of substrate that is phosphorylated in the presence of the protein kinase, wherein increased phosphorylation of the substrate in the presence of the protein kinase and the test agonist compound relative to phosphorylation in the absence of the test agonist compound is indicative of enhancement; and (c) identifying the test compound as an agonist of phosphorylation if increased phosphorylation is observed.

These methods of identifying a modulator of a protein kinase are made possible by the present invention. The present invention facilitates the identification of sequence requirements of substrates that are phosphorylated in the presence of a protein kinase of interest. This method will find particular utility in investigating newly isolated and/or identified protein kinases. Thus, the present invention allows the development of modulators by providing a known substrate for a given protein kinase that can be used in an assay where the effectiveness of a test compound can be judged.

In another aspect of the present invention, the method can be useful in computer-based structural refinement of candidate protein kinase modulators. For example, following the initial design and assay of a suspected modulator (which can be based on observed binding trends of a peptide substrate of the present invention), multiple rounds of optimization can be performed on a suspected modulator. This process can be followed by gradual structural changes of the modulator.

Although modulators can be designed by standard screening methods known to those of skill in the art upon contemplation of the present disclosure, computer software can be of particular help in the design of a modulator and can be based on the general sequence trends and consequent structural predictions of a protein kinase substrate identified by the present invention. Useful programs for designing and modeling a modulator and connecting individual chemical entities or fragments selected for inclusion in a contemplated modulator include:
1. CAVEAT^{TM} program (Bartlett et al., (1989) *Special Pub. Royal Chem. Soc.* 78: 182-96), which is available from the University of California, Berkeley, California;
2. 3D Database systems, such as MACCS-3D^{TM} system program, which is available from MDL Information Systems, San Leandro, California. This area is reviewed in Martin, (1992) *J. Med. Chem.* 35: 2145-54;
3. HOOK^{TM} program (Eisen et al., (1994). *Proteins* 19: 199-221), which is available from Molecular Simulations, Inc., San Diego, California; and
4. INSIGHT II® program, which is available from Molecular Simulations, Inc., San Diego, California.

These programs can be used, on a computer system appropriate for computer modeling techniques, to construct a peptide sequence, to modify a known peptide sequence by replacing amino acids or to modify a known peptide sequence by introducing modifications to the side chains and peptide bonds of the sequence. These programs can also be employed to design and evaluate small molecule modulators. In this way, many candidate modulators can be eliminated before they are synthesized, thus saving researchers time and resources.

Once a suspected modulator has been designed or selected by the above methods (which can be based on information about a known protein kinase substrate identified by the present invention), the efficiency with which that compound modulates phosphorylation can be assessed and optimized via computational evaluation. In this way, many candidate modulators can be eliminated without having to synthesize and evaluate each and every compound. For example, a candidate modulator that has been designed or selected to function as a protein kinase modulator should also preferably traverse a volume not overlapping that occupied by the binding site when it is bound to its native ligand. Candidate modulators that do not meet this criterion can be eliminated without having to synthesize and evaluate these modulators.

Protein kinase substrates identified using a method of the present invention can be analyzed to determine the various geometric and spatial configurations for an effective interaction with a protein kinase. Additionally, an effective protein kinase modulator or protein kinase substrate should preferably demonstrate a relatively small difference in energy between its bound and free states (i.e., a small deformation energy of binding). Thus, the most efficient protein kinase modulators should preferably be designed with a deformation energy of binding of not greater than about 10 kcal/mole, and preferably, not greater than 7 kcal/mole. It is possible for protein kinase modulators to interact with the protein kinase in more than one conformation that is similar in overall binding energy. In those cases, the deformation energy of binding can be taken to be the difference between the energy of the free compound and the average energy of the conformations observed when the modulator binds to the polypeptide.

A peptide designed or selected as being phosphorylated in the presence of a protein kinase can be further computationally optimized so that in its bound state it preferably lacks repulsive electrostatic interaction with the target polypeptide. Such non-complementary (e.g., electrostatic) interactions include repulsive charge-charge, dipole-dipole and charge-dipole interactions. Specifically, the sum of all electrostatic interactions between the modulator or peptide when the modulator or peptide is bound to a protein kinase preferably make a neutral or favorable contribution to the enthalpy of binding.

Specific computer software is available in the art to evaluate compound deformation energy and electrostatic interaction. Examples of programs designed for such uses include:
1. GAUSSIAN 98™, which is available from Gaussian, Inc., Pittsburgh, Pennsylvania;
2. AMBER^{TM} program, version 6.0, which is available from the University of California at San Francisco;
3. QUANTA^{TM} program, which is available from Molecular Simulations, Inc., San Diego, California;
4. CHARMm® program, which is available from Molecular Simulations, Inc., San Diego, California; and
5. INSIGHT II® program, which is available from Molecular Simulations, Inc., San Diego, California.

These programs can be implemented using a suitable computer system. Other hardware systems and software packages will be apparent to those skilled in the art after review of the disclosure of the present invention presented herein.

Once a protein kinase modulating compound, a peptide substrate, or a phosphorylated peptide substrate has been optimally selected and/or designed, preferably as described above, substitutions can then be,made in some of its atoms or side groups in order to improve or modify its binding properties. Generally, initial substitutions are conservative, i.e., the replacement group will have about the same size, shape, hydrophobicity and charge as the original group. It should, of course, be understood that components known in the art to alter conformation should be avoided. Such substituted chemical compounds can then be analyzed using the same computer-based approaches described in detail above.

### IV.B. Identification of Unknown Natural Protein Kinase Substrates

The natural substrates for many protein kinases are unknown. Indeed, although many protein kinases are known to catalyze the phosphorylation of substrates containing certain amino acids (notably tyrosine, serine and threonine residues), the true natural substrates of these protein kinases are not known. The present invention provides for the identification of putative natural substrates.

In accordance with the present invention, to identify an unascertained natural protein kinase substrate, the following assay method is preferably employed. Initially, a plurality of peptide sequences can be prepared and provided, as described in section III above. A suitable kinase reaction can then be performed and the peptides separated from the assay mixture components using the size-based chromatographic methods disclosed herein above and incorporated here by reference. Peptides that were phosphorylated (i.e. phosphopeptides) can then be separated from those sequences that were not phosphorylated using gallium-based metal affinity chromatography, disclosed herein above and incorporated here by reference. Isolated phosphopeptides can then be sequenced and an amino acid sequence profile (i.e. a preferred amino acid sequence) generated. The preferred amino acid sequence profile can then be analyzed to determine a new subset of peptides predicted to be phosphorylated in the presence of a protein kinase. This new subset takes into account the frequency at which each amino acid appears at each position in the peptide sequence.

An optimization procedure can subsequently be employed. In one optimization procedure, a designed subset of peptides is synthesized using standard peptide synthesis techniques and each peptide in the subset is tested for the degree to which it is phosphorylated. This second phosphorylation assay identifies those peptides that are most preferentially phosphorylated in the presence of the protein kinase under investigation. Any observed ambiguities or uncertainties can be resolved by performing multiple rounds of the disclosed optimization procedure. In this way, an amino acid sequence profile of a peptide substrate can be used to identify like sequences in putative natural protein kinase substrates.

The present inventive method can also be used to identify a short sequence, which can be a component of a larger sequence, which is recognized by a protein kinase. In this case, such a short sequence can be considered a "binding motif". A substrate of a protein kinase can thus comprise a protein or other moiety that is larger in size and sequence than a substrate or 'binding motif' identified using the present invention. Additional steps can be employed in order to determine a natural substrate of a given protein kinase. Such steps can include searching databases for proteins that comprise a substrate or binding motif identified by the present invention, or by performing alignment studies based on an identified substrate or binding motif (for example, using a "blastp" search which can be performed online at the NCBI home page (http://www.ncbi.nlm.nih.gov)).

### IV.C. Method of Designing a Protein Kinase Substrate

In another aspect of the present invention, the methods disclosed herein can be employed to design a substrate for a protein kinase. The general approach to this application of the present invention comprises starting with a substrate, modifying it and identifying the effect of the modification. Proceeding through the steps of a preferred embodiment of this application of the present inventive methods, first unmodified preferred peptide substrates are identified by (a) incubating a protein kinase with a peptide library in the presence of kinase reaction components, the library comprising library members; (b) separating library members from the kinase reaction components using size exclusion chromatography to give a pool of phosphopeptides and unphosphorylated peptides; (c) contacting the pool with immobilized gallium ions to form chelated phosphopeptides; (d) eluting chelated phosphopeptides away from the gallium ions to give eluted phosphopeptides; and (e) sequencing the eluted phosphopeptides. SEQ ID NOs: 1-10 disclose preferred peptide substrates identified as such by employing the methods of the present invention.

Next, a degree of phosphorylation of the provided unmodified preferred peptide substrate (i.e. a substrate comprising a sequence of SEQ ID NOs: 1-10) in an assay measuring protein kinase activities. This degree constitutes a reference point against which the effect of modifications to a sequence can be gauged.

After ascertaining a degree of phosphorylation of a provided unmodified peptide substrate, a chemical modification of the preferred peptide substrate is selected. The chemical modification is selected based on criteria contributing to a prediction that an interaction between the protein kinase and the unmodified preferred peptide substrate is to be modulated by the chemical modification. That is, a selected chemical modification preferably is predicted to modulate an interaction between the protein kinase and the unmodified peptide substrate. Such a prediction can be made based on a variety of criteria. Those of skill in the art, after consideration of the present disclosure, will recognize criteria that can be implicated in the modulation of an interaction. As an aid in selecting a suitable chemical modification, various computer-based modeling techniques can be employed. For example, using a program such as the INSIGHT II® program (Molecular Simulations, Inc., San Diego, California), chemical modifications such as replacement of amino acids, modification of peptide bonds and derivatization of the amino acids of a substrate can be modeled before they are synthesized. In this way, a chemical modification of a substrate can be rationally designed and preliminarily evaluated before any laboratory work is performed.

Following the selection of a suitable chemical modification, the chemical modification is performed on the unmodified preferred, peptide substrate to form a modified preferred peptide substrate. The actual chemistry of the modification is determined by the nature of the modification itself. Chemical modifications are designed with the integrity of the subject substrate in mind. That is, appropriate chemical modifications take into account the fact that the substrate is a peptide, and thus, appropriate synthetic techniques are designed accordingly.

After performing a chemical modification, the effect of the modification is determined experimentally. A determination of the effect of the modification is made by contacting the modified preferred peptide substrate with the protein kinase. Suitable reaction conditions are disclosed herein and will be apparent to those of skill in the art upon review of the disclosure of the present invention set forth herein. Suitable reaction conditions take into account buffer requirements, cofactor requirements, temperature requirements, incubation time, etc. A degree of phosphorylation of the modified preferred peptide substrate is then quantified, in an assay measuring protein kinase activity. The degree of phosphorylation is preferably determined using the same assay procedures as were employed in quantifying the degree of phosphorylation of the unmodified peptide substrate. By employing the same assay methodology, the number of variables that might contribute to any observed modulation of phosphorylation is minimized, and any observed modulation of the phosphorylation can be attributed to the effect of the chemical modification, rather than the assay conditions.

Next, the activity of the protein kinase in the presence of the modified preferred peptide substrate with the activity of the protein kinase in the presence of the unmodified preferred peptide substrate are compared. In this way, the effect of the chemical modification can be assessed. For example, a greater degree of observed phosphorylation can be indicative of a positive effect of the chemical modification, and thus, indicative of a more preferred (i.e. better) substrate for the protein kinase. A lesser degree of observed phosphorylation can be indicative of an undesired effect of a chemical modification, and thus, indicative of a lesser preferred substrate.

The above steps can be repeated a desired number of times to achieve a desired substrate property. Repeating the process of designing a chemical modification, performing the modification, evaluating the modification can lead to a more preferred substrate. This rational approach to designing and evaluating the effect of chemical modifications on a substrate can be employed to design a substrate for a protein kinase that is more preferred than the unmodified substrate that served as the starting point for the substrate design process.

### IV.D. Method of Designing a Modulator for a Protein Kinase

In yet another aspect of the present invention, a modulator for a protein kinase can be designed. The modulator can comprise a peptide. In a preferred embodiment, the method comprises performing the steps of identifying a preferred peptide substrate for a protein kinase. Thus, a peptide library can be a starting point for the disclosed method of designing a modulator for a protein kinase. Summarizing the methods presented herein for identifying a preferred peptide substrate, a protein kinase is incubated with a peptide library in the presence of kinase reaction components, the library comprising library members. The library members are separated from the kinase reaction components using size exclusion chromatography to give a pool of phosphopeptides and unphosphorylated peptides. The pool is contacted with immobilized gallium ions to form chelated phosphopeptides. Chelated phosphopeptides are eluted then away from the gallium ions to give eluted phosphopeptides and the eluted phosphopeptides are sequenced to elucidate a preferred amino acid sequence of a preferred peptide substrate for a protein kinase. These steps can be employed to identify one or more preferred peptide substrates in a peptide library, wherein the preferred peptide substrates are phosphorylated. Protein kinase activity is quantifed in an assay for a degree to which the unmodified phosphorylated preferred peptide substrate comprising a preferred amino acid sequence modulates the activity of a protein kinase. The term "modulating" thus includes but is not limited to blocking or inhibiting kinase activity.

The following steps continue with a method by which a modulator can be designed. A chemical modification of a phosphorylated preferred peptide substrate comprising a preferred amino acid sequence wherein the interaction between the protein kinase and the phosphorylated preferred peptide substrate is predicted to be modulated by the chemical modification is selected. As noted above in section IV.B., various computer programs can be of assistance when designing a chemical modification of a phosphorylated preferred peptide substrate. For example, amino acids can be replaced or modified using the INSIGHT II® program (Molecular Simulations, Inc., San Diego, California) or other suitable modeling program. These programs can also facilitate the design and evaluation of a small molecule modulator. An advantage of computer-based design of a contemplated chemical modification is the ability to qualitatively and quantitatively evaluate the effect of the chemical modification before the modification is performed in the laboratory. Contemplated modifications can be preliminarily screened before they are made, and those modifications that might lead to undesired results can be eliminated.

Next, a selected chemical modification is performed on the phosphorylated preferred peptide substrate. When the chemical modification comprises a replacement of one or more amino acids, the modification can be conveniently performed by employing an automated peptide synthesizer. Often automated peptide synthesizers can be programmed to perform various modifications as a step in the synthesis of a peptide. Alternatively, a chemical modification can be performed using organic and biochemical techniques and protocols. Reaction protocols and conditions should be selected so as to preserve the integrity of a subject phosphorylated peptide substrate. It will be recognized that a chemical modification can be, but is not required to be, performed on the amino acid residue of the phosphorylated preferred peptide substrate that was phosphorylated, by which the peptide was identified as a substrate. When the chemical modification is a substitution of one amino acid (including the residue that was phosphorylated, thereby identifying the peptide as a substrate) for another amino acid, the substitution will preferably, but not necessarily, be a conservative substitution. It should be noted .that the modification of the phosphorylated residue can result in a residue expressing an altered phosphorylation state.

Protein kinase activity is quantifed in an assay for a degree to which the modified phosphorylated preferred peptide substrate comprising a preferred amino acid sequence modulates the activity of a protein kinase. The extent of phosphorylation of a substrate is then quantified in an assay for protein kinase activity and a degree to which the modified phosphorylated preferred peptide substrate modulates the phosphorylation of a substrate in the presence of the protein kinase is identified. The assay preferably comprises the same reaction conditions as those used to assess the degree of phosphorylation of the unmodified substrate. By adhering to this approach, researchers can have a higher degree of confidence that any observed effect is due to the presence of the chemical modification and not an effect associated with variations in assay conditions between the two quantitative determinations.

Finally, the activity of the protein kinase in the presence of the modified phosphorylated preferred peptide substrate with the activity of the protein kinase in the presence of the unmodified phosphorylated preferred peptide substrate is compared. This comparison can form the basis of a determination of the effect of a selected chemical modification on the degree of modulation of phosphorylation activity in the presence of the kinase.

### Laboratory Examples

The following Laboratory Examples have been included to illustrate preferred modes of the invention. Certain aspects of the following Laboratory Examples are described in terms of techniques and procedures found or contemplated by the present inventors to work well in the practice of the invention. These Laboratory Examples are exemplified through the use of standard laboratory practices of the inventors. In light of the present disclosure and the general level of skill in the art, those of skill will appreciate that the following Laboratory Examples are intended to be exemplary only and that numerous changes, modifications and alterations can be employed without departing from the spirit and scope of the invention.

### Laboratory Example 1

### Isolation of Peptides by Size-Exclusion Chromatoaraphy

It was determined experimentally that at least about 90% of the total amount of a peptide library that is applied to the size-exclusion chromatography column is recovered in fractions that are collected for lyophilization. This determination was made by injecting a known amount of peptide library, as determined by amino acid analysis, onto the size-exclusion column and then determining by amino acid analysis the amount of peptide library present in the resulting peptide-containing fractions. The amino acid composition of the recovered material was the same as that of the starting material, indicating that there was no selective purification of members of the peptide library.

Within fractions containing peptides isolated from the other components of the protein kinase reaction, nearly all of the measured radioactivity present can be attributed to phosphopeptides (i.e., peptides from the peptide library that were recognized by the protein kinase of interest and, consequently, phosphorylated). It was determined by isolating a peptide library from a protein kinase reaction containing no protein kinase that only 0.1% of the total amount of ATP present in the protein kinase reaction ends up in the peptide fractions, as indicated by the presence of radioactivity from [γ-³³P]ATP. When phosphopeptides and unphosphorylated peptides are isolated and collected into fractions from a protein kinase reaction containing a protein kinase, the measured radioactivity in the resulting fractions is used to calculate the percentage of the original peptide library that was phosphorylated in the presence of the kinase. In general, the results of this calculation correspond well to a calculation based on an analysis using phosphocellose filter binding.

### Laboratory Example 2

### Interpretation of Phosphopeptide Sequence Data

The sample of purified phosphopeptides acquired in Laboratory Example 1 using gallium-based metal affinity chromatography was subjected to Edman sequencing using a Hewlett-Packard G1005A Protein Sequencer (Hewlett-Packard Co., Palo Alto, California). This sequencer offers the advantage of direct application of the entire sample volume without the need for a desalting or concentrating step prior to the loading of peptides onto the sequencing support, thus eliminating the potential for sample loss. The sample was applied directly to a Hewlett-Packard reverse phase support for automated Edman sequencing, and the peptides were sequenced using 3.5 chemistry with Routine 3.5 cycles. On-line phenylthiohydantoin (PTH) amino acid analysis was performed using an HP1090A Liquid Chromatograph (Hewlett-Packard Co., Palo Alto, California) with a HYPERSIL^{TM} C18 column (Thermo Hypersil Ltd., Cheshire, United Kingdom) (200mm x 2 mm inner diameter), which was packed by Column Engineering Inc., of Ontario, California. PTH amino acid yields at the individual variable positions of the peptide sequences (cycles 3-6 and cycles 8-11) were quantitated (picomole amounts) prior to and following gallium-affinity chromatography. For each variable position in each sequenced sample, a ratio (R) was calculated comparing the yield of each amino acid versus the total yield of all amino acids at that position. The ratio for the sample following gallium-affinity chromatography (R1) was compared to the ratio for the sample prior to gallium affinity chromatography (R2) to derive a "preference value" (R1/R2) for each amino acid at each variable position. The resulting data for an individual variable position is presented as a histogram in Figure 1. The amino acids with the highest preference values at each variable position of the peptide library were combined to form the preferred amino acid sequences of preferred peptide substrates that were synthesized and kinetically evaluated as protein kinase substrates.

In some cases, it can be advantageous to account for the amount of unphosphorylated peptides that are eluted from the gallium-based metal affinity chromatography column along with the phosphorylated peptides. A situation where this type of analysis can be most appropriate includes, but is not necessarily limited to, when the amount of peptide library that is phosphorylated is low, i.e. approximately 0.5 percent or less. In order to do this type of analysis a mock protein kinase catalyzed reaction is run in the presence of no protein kinase. This sample is then processed in the exact same way as the reaction containing protein kinase as described herein. The resulting peptide sequencing data can then be analyzed as described by Songyang and Cantley (Zhou Songyang and Lewis C. Cantley, (1998) *Methods Mol. Biol.* 87: 87-98) to identify the preferred amino acid sequences of preferred peptide substrates.

### Laboratory Example 3

### Determination of Preferred Peptide Substrate Sequences for ErbB-2

A peptide library comprising a tyrosine at amino acid position 7 was used to identify preferred peptide substrate sequences for the tyrosine kinase ErbB-2. Conditions were chosen under which the phosphorylation of approximately 1 % of the peptide library was catalyzed by the kinase in a total reaction volume of 0.75 mL. The phosphorylation reaction was halted by the addition of 46 µL concentrated glacial acetic acid. A portion of this halted reaction (0.3 mL) was subjected to size exclusion chromatography and the resulting fractions containing unphosphorylated peptides and phosphopeptides were pooled and lyophilized. The lyophilized sample was resuspended in 0.8 mL of 50 mM MES, pH 5.5, 1 M NaCI, and 0.75 mL of this resuspended sample was subjected to gallium-based metal affinity chromatography as described in the present disclosure. A summary of the amino acids most preferred by ErbB-2 in each variable position is provided in Table 1, along with the corresponding "preference values". This data is an example from one individual experiment and does not represent ambiguity and variability that is present from experiment to experiment. Confidence in the derived sequence of a preferred peptide substrate is gained through multiple experiments and by an enzyme kinetic analysis comparing several slightly different discrete peptides whose sequences were derived using the disclosed method.

**TABLE 1**

| A Summary Of The Amino Acids Most Preferred by ErbB-2 In Each Variable Position | | | | | | | | |
|---|---|---|---|---|---|---|---|---|
| Amino Acid Position Relative to Lone Tyrosine Residue | | | | | | | | |
| -4 | -3 | -2 | -1 | 0 | 1 | 2 | 3 | 4 |
| H(3.4) | E(2.3) | E(1.9) | E(1.9) | Y | H(3.2) | F(2.3) | F(3.0) | F(2.8) |
| E(1.9) | H(1.7) | H(1.6) | 1(1.7) | | F(2.5) | H(1.9) | 1(1.8) | L(1.5) |
| A(1.3) | D(1.2) | D(1.4) | H(1.5) | | 1(1.9) | E(1.5) | L(1.5) | H(1.3) |
| | | | | | | 1(1.5) | | 1(1.3) |

### Laboratory Example 4

### Kinetic Evaluation of Peptide Substrates

Peptide sequences identified as preferred substrates for ErbB-2 using the methods described in Laboratory Example 3 were synthesized. The k_{cat} and Kₘ values for each peptide were determined as follows, using the kinases ErbB-2, ErbB-4, and EGFR. This data is presented in Table 2.

Reactions were performed in 96-well polystyrene round bottom plates in a final volume of 45 µL. Reaction mixtures contained 50 mM MOPS, pH 7.5, 10 mM MnCl₂, 1 mM dithiothreitol (DTT), 200 µM ATP, 1.0 µCi [γ-³³P] ATP, and varying concentrations of peptide. The reaction was initiated by adding the purified kinase of interest and halted after 10 minutes at 23°C (room temperature) by adding 45 µL of 0.5 % phosphoric acid in water. A 75 µL aliquot of the halted reaction mixture was then transferred tb MAPH phosphocellulose filter plates (Millipore, Bedford, Massachusetts). The plates were filtered three times with 0.2 mL of 0.5 percent phosphoric acid. Scintillation cocktail (50 µL) was added to each well, and the plate was analyzed by counting in a Packard TOPCOUNT® scintillation counter (Packard Instrument Co., Meriden, Connecticut). In these assays, the concentration of ATP was greater than ten-fold higher than the Kₘ for ATP, which permitted the kinetic data to be treated as a pseudo-first-order reaction with varying peptide concentration. All experiments were performed in quadruplicate and the average value for each data point was analyzed. The Kₘ and Vₘₐₓ values were calculated by fitting the data to the Henri-Michaelis-Menten equation by non-linear regression using SIGMAPLOT® (SPSS Science, Chicago, lllinois). k_{cat} values were calculated using the Vₘₐₓ value, and are based on the protein concentration of the enzyme preparation and an estimate of purity determined from an evaluation of a Coomassie Blue stained SDS-polyacrylamide gel. Calculated Vₘₐₓ, Kₘ and k_{cat} data are presented in Table 2.

**TABLE 2**

| Kinetic Data Obtained for Peptide Substrates Incubated With the Kinases ErbB-2, ErbB-4 and EGFR, Wherein the ATP Concentration Was >10 x Kₘ | | | | |
|---|---|---|---|---|
| Protein Kinase | Peptide | k_{cat}(min⁻¹) | Kₘ(M) | k_{cat}/Kₘ (min⁻¹ M⁻¹) |
| ErbB-2 | 1. Biotin-Ahx-(SEQ ID NO: 12)-amide | 5.8 | 1.14 x 10⁻⁵ | 5.1 x 10⁵ |
| | 2. Biotin-Ahx-(SEQ ID NO: 13)-amide | 10.4 | 6.66 x 10⁻⁵ | 1.6 x 10⁵ |
| | 3. Biotin-Ahx-(SEQ ID NO: 14)-amide | 5.4 | 1.47 x 10⁻⁵ | 3.7 x 10⁵ |
| | 4. Biotin-Ahx-(SEQ ID NO: 15)-amide | 2.0 | 8.6 x 10⁻⁶ | 2.3 x 10⁵ |
| | 5. Biotin-Ahx-(SEQ ID NO: 16)-amide | 0.76 | 6.8 x 10⁻⁶ | 1.1 x 10⁵ |
| ErbB-4 | 1. Biotin-Ahx-(SEQ ID NO: 12)-amide | 4.1 | 8 x 10⁻⁶ | 5.1 x 10⁵ |
| | 2. Biotin-Ahx-(SEQ ID NO: 13)-amide | 4.6 | 3.76 x 10⁵ | 1.2 x 10⁵ |
| | 3. Biotin-Ahx-(SEQ ID NO: 14)-amide | 3.9 | 1.36 x 10⁻⁵ | 2.8 x 10⁵ |
| | 4. Biotin-Ahx-(SEQ ID NO: 15)-amide | 1.3 | 1.25 x 10⁻⁵ | 1.0 x 10⁵ |
| | 5. Biotin-Ahx-(SEQ ID NO: 16)-amide | 0.9 | 6 x 10⁻⁶ | 1.5 x 10⁵ |
| EGFR | 1. Biotin-Ahx-(SEQ ID NO: 12)-amide | 6.1 | 8.0 x 10⁻⁶ | 7.5 x 10⁵ |
| | 2. Biotin-Ahx-(SEQ ID NO: 13)-amide | 15.1 | 1.05 x 10⁻⁴ | 1.4 x 10⁵ |
| | 3. Biotin-Ahx-(SEQ ID NO: 14)-amide | 6.5 | 1.22 x 10⁻⁵ | 5.3 x 10⁵ |
| | 4. Biotin-Ahx-(SEQ ID NO: 15)-amide | 3.4 | 1.0 x 10⁻⁵ | 3.4 x 10⁵ |
| | 5. Biotin-Ahx-(SEQ ID NO: 16)-amide | 2.4 | 7.5 x 10⁻⁶ | 3.1 x 10⁵ |

### Laboratory Example 5

### Comparison of Kinetic Data of a Protein Kinase Substrate Identified by Employing the Present Invention With a Known Protein Kinase Substrate

In a separate experiment, the kinetic parameters of both peptide number 1 shown in Table 2, and a peptide with the sequence Biotin-Ahx-G-G-M-E-D-l-Y-F-E-F-M-G-G-K-K-K-amide (SEQ ID NO: 17), which has been previously identified as an ErbB-2 substrate (Jan et. al., (2000), *Biochemistry* 39: 9786-803), were determined and compared. In this experiment, both peptide and ATP concentrations were varied, and the resulting data was simultaneously fit to an equation representing a two-substrate enzyme reaction using SIGMAPLOT®. Data was collected using the kinases ErbB-2, ErbB-4, and EGFR. The results of this experiment are shown in Table 3 and indicate that for all three kinases tested, peptide number 1 shown in Table 2 is kinetically at least equivalent (based on k_{cat}/Kₘ values) to the known peptide substrate identified by Jan et al.

**TABLE 3**

| Kinetic Data Obtained from Peptide Substrates Incubated with the Kinases ErbB-2, ErbB-4 and EGFR, Wherein Peptide and ATP Concentrations Were Varied Simultaneously | | | | |
|---|---|---|---|---|
| Protein Kinase | Peptide | k_{cat}(min⁻¹) | Kₘ (M) | k_{cat}/Kₘ (min⁻¹ M⁻¹) |
| ErbB-2 | Biotin-Ahx-(SEQ ID NO: 12)-amide | 1.4 | 5.8 x 10⁻⁵ | 2.5 x 10⁴ |
| | Biotin-Ahx-(SEQ ID NO: 17)-amide | 2.2 | 1.57 x 10⁻⁴ | 1.4 x 10⁴ |
| ErbB-4 | Biotin-Ahx-(SEQ ID NO: 12)-amide | 6.3 | 4.1 x 10⁻⁵ | 1.5 x 10⁵ |
| | Biotin-Ahx-(SEQ ID NO: 17)-amide | 4.6 | 1.44 x 10⁻⁴ | 3.2 x 10⁴ |
| EGFR | Biotin-Ahx-(SEQ ID NO: 12)-amide | 3.4 | 1.28 x 10⁻⁴ | 2.7 x 10⁴ |
| | Biotin-Ahx-(SEQ ID NO: 17)-amide | 13.1 | 8.99 x 10⁻⁴ | 1.5 x 10⁴ |

### References

The references listed below as well as all references cited in the specification are incorporated herein by reference to the extent that they supplement, explain, provide a background for or teach methodology, techniques and/or compositions employed herein.
Aebersold et al., (1990) *Anal. Biochem.* 187: 56-65
Ausubel et al., (1992) Current Protocols in Molecular Biology, John Wylie and Sons, Inc., New York
Bartlett et al., (1989) *Special Pub. Royal Chem. Soc.* 78: 182-96
Bodanszky et al., (1976) Peptide Synthesis, Second Edition, John Wiley & Sons, Inc., New York
Dieffenbach & Dveksler, (1995) PCR Primers: A Laboratory Manual, Cold Spring Harbor Laboratory Press, Plainview, New York
Druker et al., (1996) *Nat. Med.* 2: 561-66
Eisen et al., (1994). *Proteins* 19: 199-221
Fields et al., *Int. J. Peptide Protein Res.,* 35:161-214, 1990
Glover, (ed.) (1985) DNA Cloning: A Practical Approach, MRL Press, Ltd., Oxford, U.K.
Govoni et al., (1996) *Ann. N.Y. Acad. Sci.* 777: 332-37
Grammas et al., (1995) *Neurobiol. Aging* 16: 563-69
Hagel, (1993) *J. Chromatogr.* 648:19
Hewick et al., (1981) *J. Biol. Chem.* 256: 7990-97
Hunkapillar et al., (1984) *Science* 226: 304-11
Jan et. al., (2000), *Biochemistry* 39: 9786-803
Janson & Rydén (eds), (1998) Protein Purification: Principles, High Resolution Methods, and Applications (2^{nd} ed.), Wiley-Liss, New York
Konigsberg & Steinman, (1977) in The Proteins (3^{rd} ed.) (Neurath & Hill, eds.) 3: 1-178
Martin, (1992) *J. Med. Chem.* 35: 2145-54
McOmie, (1973) Protective Groups in Organic Chemistry, Plenum Press, New York
Meienhofer, (1983) Hormonal Proteins and Peptides, Vol. 2, p. 46, Academic Press, New York
Merrifield, *Adv Enzymol,* 32: 221-96, 1969
Posewitz & Tempst, (1999) *Anal. Chem.* 71(14): 2883-92
Sambrook et al., (eds.) (1989) *Molecular Cloning,* Cold Spring Harbor Laboratory Press, Cold Spring Harbor, New York
Sanpei et al., (1995) *Biochem. Biophys. Res. Commun.* 212: 341-46
Schroder et al., (1965) The Peptides, Vol. 1, Academic Press, New York
Silhavy et al., (1984) Experiments with Gene Fusions, Cold Spring Harbor Laboratory Press, Cold Spring Harbor, New York
Sperber et al., (1995) *Neurosci. Lett.* 197: 149-53
Stein & Udenfriend, (1984) *Anal. Chem.* 136: 7-23
Steward et al., (1969) Solid Phase Peptide Synthesis, W. H. Freeman Co., San Francisco
U.S. Patent No. 4,244,946
U.S. Patent No. 5,260,203
U.S. Patent No. 5,532,167
U.S. Patent No. 6,004,757

It will be understood that various details of the invention can be changed without departing from the scope of the invention. Furthermore, the foregoing description is for the purpose of illustration only, and not for the purpose of limitation―the invention being defined by the claims.

## Claims

1. A method of elucidating a preferred amino acid sequence of a preferred peptide substrate for a protein kinase, the method comprising:
(a) incubating a protein kinase with a peptide library in the presence of kinase reaction components, the library comprising library members;
(b) separating library members from the kinase reaction components using size exclusion chromatography to give a pool of phosphopeptides and unphosphorylated peptides;
(c) contacting the pool with immobilized gallium ions to form chelated phosphopeptides;
(d) eluting chelated phosphopeptides away from the gallium ions to give eluted phosphopeptides; and
(e) sequencing the eluted phosphopeptides, whereby a preferred amino acid sequence of a preferred peptide substrate for a protein kinase is elucidated.

2. A method of identifying a compound that modulates the protein kinase catalyzed phosphorylation of a preferred peptide substrate, the method comprising:
(a) incubating a protein kinase with a peptide library in the presence of kinase reaction components, the library comprising library members;
(b) separating library members from the kinase reaction components using size exclusion chromatography to give a pool of phosphopeptides and unphosphorylated peptides;
(c) contacting the pool with immobilized gallium ions to form chelated phosphopeptides;
(d) eluting chelated phosphopeptides away from the gallium ions to give eluted phosphopeptides;
(e) sequencing the eluted phosphopeptides to elucidate a preferred amino acid sequence of a preferred peptide substrate for a protein kinase;
(f) incubating a protein kinase with a preferred peptide substrate comprising the preferred amino acid sequence in the presence and absence of a test modulator compound; and
(g) determining an amount of preferred peptide substrate that is phosphorylated in the presence of the protein kinase, wherein a difference in phosphorylation of the preferred peptide substrate in the presence of the test modulator compound relative to phosphorylation of the preferred peptide substrate in the absence of the test modulator compound is indicative of modulation, whereby a compound that modulates the protein kinase catalyzed phosphorylation of a preferred peptide substrate is identified.

3. A method of designing a modulator for a protein kinase, the method comprising:
(a) incubating a protein kinase with a peptide library in the presence of kinase reaction components, the library comprising library members;
(b) separating library members from the kinase reaction components using size exclusion chromatography to give a pool of phosphopeptides and unphosphorylated peptides;
(c) contacting the pool with immobilized gallium ions to form chelated phosphopeptides;
(d) eluting chelated phosphopeptides away from the gallium ions to give eluted phosphopeptides;
(e) sequencing the eluted phosphopeptides to elucidate a preferred amino acid sequence of a preferred peptide substrate for a protein kinase, wherein the preferred peptide substrate is phosphorylated;
(f) quantifying in an assay for protein kinase activity a degree to which the phosphorylated preferred peptide substrate comprising a preferred amino acid sequence modulates the activity of the protein kinase;
(g) selecting a chemical modification of the phosphorylated preferred peptide substrate comprising a preferred amino acid sequence wherein the interaction between the protein kinase and the phosphorylated preferred peptide substrate is predicted to be modulated by the chemical modification;
(h) performing the chemical modification on the phosphorylated preferred peptide substrate;
(i) contacting the modified phosphorylated preferred peptide substrate with the protein kinase;
(j) quantifying in a biological assay for protein kinase activity a degree to which the modified phosphorylated preferred peptide substrate modulates the biological activity of the protein kinase; and
(k) comparing the activity of the protein kinase in the presence of the modified phosphorylated preferred peptide substrate with the activity of the protein kinase in the absence of the modified phosphorylated preferred peptide substrate, whereby a modulator for a protein kinase is designed.

4. A method of elucidating a preferred amino acid sequence of a preferred peptide substrate for a protein kinase, the method comprising:
(a) incubating a protein kinase with a peptide library in the presence of kinase reaction components, the library comprising library members having one or more variant positions;
(b) separating library members from the kinase reaction components using size exclusion chromatography to give a pool of phosphopeptides and unphosphorylated peptides;
(c) contacting the pool with immobilized gallium ions to form chelated phosphopeptides;
(d) eluting chelated phosphopeptides away from the gallium ions to give eluted phosphopeptides;
(e) sequencing the eluted phosphopeptides;
(f) fixing a residue at one or more variant positions in a library member; and
(g) repeating steps (a) through (d) a desired number of times, whereby a preferred amino acid sequence of a preferred peptide substrate for a protein kinase is elucidated.

5. The method of any one of claims 1 to 4 wherein the protein kinase is selected from the group consisting of serine/threonine kinases and tyrosine kinases.

6. The method of any one of claims 1 to 4 wherein the library or preferred peptide substrate is cleavably disposed on a support.

7. The method of any one of claims 1 to 4 wherein the library or preferred peptide substrate is cleavably disposed on the surface of a phage.

8. The method of any one of claims 1 to 3 wherein the library members or preferred peptide substrate are 15-mers, optionally amidated at the C-terminus.

9. The method of claim 8, wherein the 15-mers have a sequence selected from the group consisting of M-A-X-X-X-X-Y-X-X-X-X-A-K-K-K (SEQ ID NO: 1), M-A-X-X-X-X-S-X-X-X-X-A-K-K-K (SEQ ID NO: 2), M-A-X-X-X-X-T-X-X-X-X-A-K-K-K (SEQ ID NO: 3), M-A-X-X-X-X-S-P-X-X-X-A-K-K-K (SEQ ID NO: 4), M-A-X-X-X-X-T-P-X-X-X-A-K-K-K (SEQ ID NO: 5), R-A-X-X-X-X-Y-X-X-X-X-A-K-K-K (SEQ ID NO: 6), R-A-X-X-X-X-S-X-X-X-X-A-K-K-K (SEQ ID NO: 7), R-A-X-X-X-X-T-X-X-X-X-A-K-K-K (SEQ ID NO: 8), R-A-X-X-X-X-S-P-X-X-X-A-K-K-K (SEQ ID NO: 9), and R-A-X-X-X-X-T-P-X-X-X-A-K-K-K (SEQ ID NO: 10), wherein X independently represents any amino acid.

10. The method of claim 9, wherein X is independent and is selected from the group consisting of A, R, N, D, E, Q, G, H, I, L, K, M, F, P and V.

11. The method of claim 4 wherein the library members are 15-mers, optionally amidated at the C-terminus.

12. The method of claim 11, wherein the 15-mers have a sequence selected from the group consisting of M-A-X-X-X-X-Y-X-X-X-X-A-K-K-K (SEQ ID NO: 1), M-A-X-X-X-X-S-X-X-X-X-A-K-K-K (SEQ ID NO: 2), M-A-X-X-X-X-T-X-X-X-X-A-K-K-K (SEQ ID NO: 3), M-A-X-X-X-X-S-P-X-X-X-A-K-K-K (SEQ ID NO: 4), M-A-X-X-X-X-T-P-X-X-X-A-K-K-K (SEQ ID NO: 5), R-A-X-X-X-X-Y-X-X-X-X-A-K-K-K (SEQ ID NO: 6), R-A-X-X-X-X-S-X-X-X-X-A-K-K-K (SEQ ID NO: 7), R-A-X-X-X-X-T-X-X-X-X-A-K-K-K (SEQ ID NO: 8), R-A-X-X-X-X-S-P-X-X-X-A-K-K-K (SEQ ID NO: 9), and R-A-X-X-X-X-T-P-X-X-X-A-K-K-K (SEQ ID NO: 10), wherein X independently represents any amino acid.

13. The method of claim 12, wherein X is independent and is selected from the group consisting of A, R, N, D, E, Q, G, H, I, L, K, M, F, P, V, Y, S, T, phosphotyrosine, phosphoserine and phosphothreonine.

14. The method of any one of claims 1 to 4, further comprising lyophilizing and resuspending the library members before incubating with a protein kinase.

15. The method of claim 2, wherein the preferred peptide substrate is an eluted phosphopeptide.

16. The method of claim 3, wherein the chemical modification is phosphorylation.

17. The method of claim 3, wherein steps (a) through (k) are repeated a desired number of times.

18. The method of claim 4, wherein the method is repeated for each variant position in a library member.

19. A method of designing a substrate for a protein kinase, the method comprising:
(a) providing an unmodified preferred peptide substrate selected from the group consisting of SEQ ID NOs. 1-10;
(b) quantifying phosphorylation of the unmodified preferred peptide substrate in an assay measuring protein kinase activity;
(c) selecting a chemical modification of the preferred peptide substrate wherein an interaction between the protein kinase and the unmodified preferred peptide substrate is predicted to be modulated by the chemical modification;
(d) performing the chemical modification on the unmodified preferred peptide substrate to form a modified preferred peptide substrate;
(e) contacting the modified preferred peptide substrate with the protein kinase;
(f) quantifying phosphorylation of the modified preferred peptide substrate, in an assay measuring protein kinase activity;
(g) comparing the activities of the protein kinase in the presence of the modified preferred peptide substrate with the activity of the protein kinase in the presence of the unmodified preferred peptide substrate; and
(h) repeating steps (c) through (g) a desired number of times to achieve a desired substrate property, whereby a substrate for a protein kinase is designed.

20. The method of claim 19, wherein the unmodified preferred peptide substrate is amidated on the C-terminus.

21. The method of claim 19, wherein the chemical modification is phosphorylation.
